# EUROPÄISCHE PATENTSCHRIFT

(11) **EP 3 630 048 B1**
(45) Veröffentlichungstag und Bekanntmachung des Hinweises auf die Patenterteilung: **07.02.2024**
(21) Anmeldenummer: 17731816.9
(22) Anmeldetag: 01.06.2017
(51) Int. Cl.: A23L 27/00, A23L 27/20, A61K 8/33, A61Q 19/00, C11B 9/00

(54) **VERFAHREN ZUR HERSTELLUNG EINER AROMAMISCHUNG ENTHALTEND UNGESÄTTIGTE DIENALE**
METHOD TO PRODUCE AN AROMA MIXTURE COMPRISING UNSATURATED DIENALS
PROCÉDÉ DE PRÉPARATION D'UN MÉLANGE D'ARÔMES CONTENANT DES DIÉNALS INSATURÉS

(43) Veröffentlichungstag der Anmeldung: 08.04.2020
(73) Patentinhaber: Symrise AG, 37603 Holzminden (DE)
(72) Erfinder: BACKES, Michael, 37603 Holzminden (DE); ONGOUTA, Jekaterina, 37627 Stadtoldendorf (DE); LEY, Jakob, 37603 Holzminden (DE); VÖSSING, Tobias, 37688 Beverungen (DE); KOPPE, Volkmar, 37671 Höxter-Stahle (DE); KOCH, Jens, 37632 Eschershausen (DE)
(74) Vertreter: Eisenführ Speiser
(86) Internationale Anmeldenummer: PCT/EP2017/063368
(87) Internationale Veröffentlichungsnummer: WO 2018/219467

(56) Entgegenhaltungen:
- EP-A1- 0 911 414
- EP-A2- 1 336 659
- EP-B1- 1 244 364
- WO-A2-02/103023

## Beschreibung

Die vorliegende Erfindung betrifft primär ein Verfahren zur Herstellung einer Aromamischung enthaltend ungesättigte Dienale. Die Erfindung betrifft zudem Aromamischungen hergestellt durch ein erfindungsgemäßes Verfahren und Zubereitungen oder Halbfertigwaren zur Herstellung besagter Zubereitungen, umfassend erfindungsgemäße Aromamischungen. Ein weiterer Aspekt der vorliegenden Erfindung betrifft die Verwendung einer erfindungsgemäßen Aromamischung zur Aromatisierung einer Zubereitung, vorzugsweise einer der Ernährung oder dem Genuss dienenden Zubereitung, oder einer Halbfertigware zur Herstellung einer solchen Zubereitung.

Weitere Aspekte und bevorzugte Ausgestaltungen der vorliegenden Erfindung ergeben sich aus den nachfolgenden Ausführungen, den beigefügten Beispielen und insbesondere den beigefügten Patentansprüchen.

Die gezielte Herstellung und Verwendung von Aromamischungen bestimmter Geschmacksrichtungen, die lebensmittelrechtlich kompatibel sind, ist Gegenstand ständiger Forschungsanstrengungen.

Zu den für die Aromenindustrie besonders interessanten Geschmacksstoffen zählen dabei beispielsweise mehrfach ungesättigte Aldehyde, insbesondere 2E,4E-Decadienal und 2E,4E-Nonadienal sowie deren Mischungen mit ihren jeweilig entsprechenden stereoisomeren Formen ((2Z,4E), (2E,4Z) und (2Z,4Z)). 2,4-Nonadienal (FEMA 3212, EFSA 05.071 und 05.194) und 2,4-Decadienal (FEMA 3135; EFSA 05.081 und 05.140) selbst sind bekannte Aromastoffe, die in vielen Aromakompositionen Verwendung finden. Mischungen enthaltend 2,4-Nonadienal oder 2,4-Decadienal zeichnen sich in vielen Anwendungen durch einen besonders natürlichen Fleischgeschmack aus (besonders nach Huhn und Rind), finden aber auch Verwendung in Frucht- und Gemüsearomen (z.B. Birne-, Spargel- bzw. Tomatenaroma) sowie zur Aromatisierung von Spirituosen (z.B. von Whiskey, Tequila, Brandy).

Die Herstellung von Aromastoffen enthaltend mehrfach ungesättigte Aldehyde erfolgt meist vollsynthetisch aus kleineren Bauteilen mittels Kupplungsreaktionen oder durch Aufkonzentrierung von Nebenströmen der Raffination von Speiseölen. In diesen Ölen werden gesättigte und ungesättigte Aldehyde durch (teilweise enzymatische) Reaktionen in Verbindung mit Luft in Spuren gebildet und sind oft für ein ranziges Fehlaroma verantwortlich und werden aus diesem Grund vorzugsweise entfernt.

Für Aromakompositionen stellen diese Aldehyde jedoch einen wertvollen Rohstoff dar, der allerdings nicht immer oder nur zu sehr hohen Preisen verfügbar ist.

Enzymatische Verfahren zur Herstellung von Aromamischungen sind aus EP1244364 B1, WO02/103023 A2, EP1336659 A2 und EP0911414 A1 vorbekannt.

Die primäre Aufgabe bestand daher darin, neue, vorzugsweise kostengünstige und einfache, Herstellungsmethoden zur Darstellung von Aromamischungen enthaltend ungesättigte Dienale zu finden.

Diese primäre gestellte Aufgabe wird erfindungsgemäß gelöst durch ein Verfahren zur Herstellung einer Aromamischung enthaltend ein oder mehrere ungesättigte(s) Dienal(e), bestehend aus oder umfassend die Schritte
a) Luftoxidation eines oder mehrerer Edukte(s) umfassend oder bestehend aus einem oder mehreren Stoff(en) ausgewählt aus der Gruppe bestehend aus Punicinsäure, Punicinsäureester, Elaeostearinsäure, Elaeostearinsäureester, Linolsäure und Linolsäureester,
b) optional Zersetzung eines Teils oder aller oder im Wesentlichen aller Peroxide in der in Schritt a) erhaltenen Mischung,
c) Aufreinigung bzw. Aufkonzentrierung des/der in Schritt a) entstandenen ungesättigten Dienals/Dienale aus bzw. in der in Schritt a) und/oder b), falls vorhanden, erhaltenen Mischung,
wobei die Luftoxidation in Schritt a) bei einer Temperatur von 70 bis 200°C, vorzugsweise von 80 bis 200°C, bevorzugt von 90 bis 170°C, weiter bevorzugt von 100 bis 130°C, durchgeführt wird.

Zur Herstellung einer Aromamischung enthaltend 2,4-Nonadienal wird/werden gemäß einer bevorzugten Ausführungsform des erfindungsgemäßen Verfahrens in Schritt a) Punicinsäure und/oder ein Punicinsäureester und/oder Elaeostearinsäure und/oder ein Elaeostearinsäureester als Edukt(e) eingesetzt.

Zur Herstellung einer Aromamischung enthaltend 2,4-Decadienal wird/werden gemäß einer weiteren bevorzugten Ausführungsform des erfindungsgemäßen Verfahrens in Schritt a) Linolsäure und/oder ein Linolsäuresäureester als Edukt eingesetzt.

Im Rahmen des vorliegenden Textes steht der Begriff "2,4-Nonadienal" für ein einzelnes Stereoisomer dieser Verbindung, also (2E,4E), (2Z,4E), (2E,4Z) oder (2Z,4Z)-Nonadienal oder für eine Mischung aus zwei oder mehr dieser stereoisomeren Formen. Das Gleiche gilt analog für 2,4-Decadienal.

Überraschenderweise hat sich herausgestellt, dass das erfindungsgemäße Verfahren insbesondere die Herstellung von 2,4-Nonadienal sowie 2,4-Decadienal in einer sensorisch akzeptablen, in vielen Fällen sogar präferierten Qualität gegenüber dem bisherigen Stand der Technik, ermöglicht.

Mit Hilfe des erfindungsgemäßen Verfahrens können vorteilhafterweise die folgenden Vorteile beobachtet bzw. erzielt werden:
- Das Verfahren ist lebensmitteltechnologisch und -rechtlich ohne Bedenken einsetzbar.
- Das Verfahren kann mehrfach wiederholt werden, um so die Ausbeute zu steigern.
- Das Verfahren erfordert keine ungewöhnlichen Verfahrensschritte oder Prozeduren, so dass es mit herkömmlichen Produktionsanlagen problemlos einsetzbar ist.
- Das Verfahren liefert ein Produkt mit komplexem Aromenprofil, welches als besonders natürlich beschrieben wird.
- Das Verfahren beruht auf dem Einsatz nachwachsender Rohstoffe ohne die Verwendung stöchiometrischer Reagenzien oder metallischer Katalysatoren.
- Das Verfahren ermöglicht eine einfache und kostengünstige Durchführung.

Unter dem Begriff "Punicinsäure" wird im Rahmen des vorliegenden Textes reine Punicinsäure ((9Z,11E,13Z)-Octadeca-9,11,13-triensäure) verstanden.

Unter dem Begriff "Punicinsäureester" werden im Rahmen des vorliegenden Textes die Ester der Punicinsäure, bevorzugt Methyl-, Ethyl- oder Hydroxyalkylester davon, verstanden. Besonders bevorzugt sind Glycerinmonoester, Glycerindiester und Glycerintriester der Punicinsäure und deren Mischungen.

Unter dem Begriff "Elaeostearinsäure" wird im Rahmen des vorliegenden Textes reine alpha-Elaeostearinsäure ((9Z,11E,13E)-Octadeca-9,11,13-triensäure) bzw. reine beta-Elaeostearinsäure ((9E,11E,13E)-Octadeca-9,11,13-triensäure) sowie deren Mischungen verstanden.

Unter dem Begriff "Elaeostearinsäureester" werden im Rahmen des vorliegenden Textes die Ester der alpha-Elaeostearinsäure bzw. beta-Elaeostearinsäure, bevorzugt deren Methyl-, Ethyl- oder Hydroxyalkylester, sowie deren Mischungen verstanden. Besonders bevorzugt sind Glycerinmonoester, Glycerindiester und Glycerintriester der Elaeostearinsäure und deren Mischungen.

Unter dem Begriff "Linolsäure" wird reine Linolsäure ((9Z,12Z)-Octadeca-9,12-diensäure) verstanden.

Unter dem Begriff "Linolsäuresäureester" werden die Ester der Linolsäure, bevorzugt deren Methyl-, Ethyl- oder Hydroxyalkylester, verstanden. Besonders bevorzugt sind Glycerinmonoester, Glycerindiester und Glycerintriester der Linolsäure und deren Mischungen.

Im Rahmen des erfindungsgemäßen Verfahrens können gemäß einer bevorzugten Ausführungsform in Schritt a) zudem auch Edukte verwendet werden, die Punicinsäure(ester), Elaeostearinsäure(ester) und/oder Linolsäure(ester) bzw. Mischungen davon enthalten. Besonders bevorzugt ist dabei die Verwendung eines Öls als Edukt, welches als wesentlichen Bestandteil der enthaltenen glyceridisch gebundenen Fettsäuren Punicinsäure, Elaeostearinsäure und/oder Linolsäure aufweist. Bevorzugt werden dabei zum Verzehr geeignete Öle eingesetzt, die 20 bis 80 Gew-% gebundener Punicinsäure, Elaeostearinsäure und/oder Linolsäure enthalten.

Im Falle von Punicinsäure- bzw. Elaeostearinsäure-haltigen Ölen sind Granatapfelkernöl, Schlangengurkenkernöl, Bittermelonenkernöl sowie Tungöl besonders zum Einsatz als Edukte in Schritt a) des erfindungsgemäßen Verfahrens geeignet. Bevorzugt ist dabei die Verwendung von Granatapfelkernöl.

Im Falle von Linolsäure-haltigen Ölen sind Traubenkernöl, Distelöl, Hanföl, Sojaöl, Weizenkeimöl, Maiskeimöl sowie Sonnenblumenöl besonders zum Einsatz als Edukte in Schritt a) des erfindungsgemäßen Verfahrens geeignet. Bevorzugt wird dabei die Verwendung von Sonnenblumenöl, mit einem möglichst hohen Anteil an Linolsäure, besonders bevorzugt mit einem Anteil an Linolsäure von ≥ 40 Gew.-%.

Gemäß einer weiteren bevorzugten Ausführungsform des erfindungsgemäßen Verfahrens wird beim Einsatz eines Öls mit einem Anteil an glyceridisch gebundener Punicinsäure, Elaeostearinsäure und/oder Linolsäure oder mit einem Anteil an anderen Estern der Punicinsäure, Elaeostearinsäure und/oder Linolsäure als Edukt vor Schritt a) zusätzlich zu der im Öl gebundenen Punicinsäure, Elaeostearinsäure und/oder Linolsäure freie ungesättigte Fettsäure, bevorzugt in einem Anteil im Bereich von 5 bis 10 Gew.-%, bezogen auf das Gesamtgewicht des/der Edukte(s), zugesetzt. Besonders bevorzugt werden in diesem Fall freie Punicinsäure, Elaeostearinsäure und/oder Linolsäure zugesetzt.

Bevorzugt ist ein erfindungsgemäßes Verfahren wie vorangehend beschrieben, wobei nach Aufreinigung bzw. Aufkonzentrierung in Schritt c) ein Rückstand erhalten wird, der eine Restmenge an Edukt(en) enthält und wobei das Verfahren zusätzlich folgenden Schritt umfasst:
d) Wiederholen, bevorzugt mehrmaliges Wiederholen, der vorangehend definierten Verfahrensschritte, wobei der bei der Aufreinigung bzw. Aufkonzentrierung in Schritt c) erhaltene Rückstand in Schritt a) eingesetzt wird.

Bevorzugt wird die Sequenz der beschriebenen Schritte des erfindungsgemäßen Verfahrens insgesamt drei- bis fünfmal, bevorzugt viermal, wiederholt.

Die (insbesondere mehrmalige) Rückführung des bei der Aufreinigung bzw. Aufkonzentrierung in Schritt c) des Verfahrens erhaltenen Rückstands, d.h. die Rückführung der im ersten Reaktionszyklus noch nicht zu ungesättigten Dienalen reagierten Edukte in der Mischung, in das Verfahren ist besonders vorteilhaft, da dadurch die Ausbeute des Verfahrens signifikant erhöht werden kann und Ausschussmaterial minimiert wird. Dies führt wiederum zu starken Kosteneinsparungen bei der Durchführung des erfindungsgemäßen Verfahrens.

Gemäß einer weiteren bevorzugten Ausführungsform des erfindungsgemäßen Verfahrens wird Schritt a) in Gegenwart eines oder mehrerer Lösungsmittel(s) durchgeführt, vorzugsweise wobei das bzw. ein oder zwei bzw. die Lösungsmittel Triacetin und/oder Triethylcitrat ist bzw. sind, besonders bevorzugt wobei insgesamt 2 bis 20 Gew.-%, weiter bevorzugt 5 bis 15 Gew.-%, jeweils bezogen auf das Gesamtgewicht des/der Edukte(s), an Lösungsmittel(n), vorzugsweise an Triacetin und/oder Triethylcitrat, eingesetzt werden.

Gemäß einer weiteren bevorzugten Ausführungsform des erfindungsgemäßen Verfahrens wird für die Luftoxidation in Schritt a) eine Luftmenge von 10 bis 1000 l/h, vorzugsweise 30 bis 500 l/h, besonders bevorzugt 40 bis 100 l/h, pro kg Edukt(e) eingesetzt.

Vorzugsweise erfolgt die Luftoxidation dabei durch Begasen mit (Umgebungs-)Luft. Der Einsatz einer Luftmenge von 10 bis 1000 I/h pro kg Edukt(e) hat sich als besonders vorteilhaft herausgestellt. Bei geringeren Luftmengen ist oftmals die Ausbeute zu gering, höhere Luftmengen führen oftmals zur Bildung von Nebenprodukten oder Zersetzung der entstehenden gewünschten Produkte.

Bevorzugt wird die Luftoxidation in Schritt a) des erfindungsgemäßen Verfahrens über einen Zeitraum von 1 bis 30 h, bevorzugt 2 bis 12 h, besonders bevorzugt von 4 bis 12 h, durchgeführt wird.

Die Durchführung der Luftoxidation in Schritt a) des erfindungsgemäßen Verfahrens über einen Zeitraum von 1 bis 30 h ist besonders vorteilhaft, da längere Reaktionsdauern oftmals zur Entstehung von unerwünschten Nebenprodukte in zu großen Mengen führen.

Gemäß einer bevorzugten Ausführungsform des erfindungsgemäßen Verfahrens wird Schritt a) über einen Zeitraum von 2 bis 12 h durchgeführt.

Gemäß einer weiteren alternativen Ausführungsform des erfindungsgemäßen Verfahrens wird Schritt a) über einen Zeitraum von 4 bis 12 h durchgeführt.

Vorzugsweise findet in Schritt b), falls vorhanden, des erfindungsgemäßen Verfahrens die Zersetzung eines Teils oder aller oder im Wesentlichen aller Peroxide in der in Schritt a) erhaltenen Mischung, bevorzugt durch Erhitzen, bevorzugt auf Temperaturen von 100 bis 200°C, statt, vorzugsweise über einen Zeitraum von 0,5 bis 5 h.

Falls die in Schritt a) des erfindungsgemäßen Verfahrens erhaltene Mischung eine Peroxidzahl von mehr als 30 meq O/kg aufweist, ist es vorteilhaft, die Peroxidzahl durch Erhitzen vor Schritt c) des Verfahrens auf weniger als 30 meq O/kg, bevorzugt weniger als 20 meq O/kg, zu reduzieren. Die Peroxidzahl wird dabei bevorzugt durch die Methode nach Wheeler bestimmt.

Die Zersetzung eines Teils oder aller oder im Wesentlichen aller Peroxide in der in Schritt a) erhaltenen Mischung vor der in Schritt c) des erfindungsgemäßen Verfahrens stattfindenden Aufreinigung bzw. Aufkonzentrierung ist aus Sicherheitsaspekten besonders vorteilhaft. Die Durchführung des Schritts b) im erfindungsgemäßen Verfahren ist des Weiteren besonders vorteilhaft, da ein solches Verfahren zu stabileren Mischungen (aus Aromensicht) und zu einem höheren Gehalt an Dienalen in der Aromamischung führt, da die Peroxide nicht über längere Zeiträume bzw. nicht erst während der Aufreinigung bzw. Aufkonzentrierung in Schritt c) des erfindungsgemäßen Verfahrens abreagieren, wenn die Mischung immer konzentrierter wird.

Gemäß einer bevorzugten Ausführungsform wird Schritt b) des erfindungsgemäßen Verfahrens bei einer Temperatur von 120 bis 140°C und/oder über einen Zeitraum von 1,5 bis 2,5 h durchgeführt.

Alternativ wird gemäß einer weiteren bevorzugten Ausführungsform Schritt b) des erfindungsgemäßen Verfahrens bei einer Temperatur von 160 bis 180°C und/oder über einen Zeitraum von 0,5 bis 1,5 h durchgeführt.

Vorzugsweise findet die Aufreinigung bzw. Aufkonzentrierung in Schritt c) des erfindungsgemäßen Verfahrens mittels Destillation, vorzugsweise mittels fraktionierter Destillation, statt.

Bevorzugt werden während der Destillation zunächst leicht flüchtige Verbindungen abgetrennt. Dies hat sich oftmals als günstig herausgestellt, da so bei vielen Anwendungen auf einfache Weise unerwünschte Fehlnoten (z.B. fruchtige und grüne Noten oder stechende Geruchseindrücke), welche durch vorhandene kurzkettige gesättigte und ungesättigte Aldehyde in der Mischung entstehen können, effektiv aus der Aromamischung entfernt werden können.

Bevorzugt ist ein einfindungsgemäßes Verfahren, wobei vor Beginn der Aufreinigung bzw. Aufkonzentrierung in Schritt c) ein oder mehrere Lösungsmittel, bevorzugt Triacetin, zugegeben wird bzw. werden, vorzugsweise in einer Gesamtmenge von 1 bis 60 Gew.-%, bevorzugt von 1 bis 40 Gew.-%, besonders bevorzugt von 2 bis 20 Gew.-%, jeweils bezogen auf das Gesamtgewicht des/der Edukte(s).

Gemäß einer bevorzugten Ausführungsform des erfindungsgemäßen Verfahrens erfolgt vor Beginn der Aufreinigung bzw. Aufkonzentrierung in Schritt c), vorzugsweise vor Beginn der Destillation, ein Waschschritt mit basischer, wässriger Lösung (z.B. einer wässrigen Natriumhydrogencarbonatlösung).

Eine solche Verfahrensführung ist besonders vorteilhaft, da durch diesen zusätzlichen Reinigungsschritt eventuell vorhandene Säuren vor der Destillation aus der Mischung entfernt werden.

Die vorliegende Erfindung betrifft gemäß einem weiteren Aspekt eine Aromamischung, hergestellt durch ein Verfahren wie vorangehend beschrieben, in der das Gewichtsverhältnis von 2E/4E-Decadienal zu 2E/4Z-Decadienal 4:1 bis 5:1 beträgt und/oder in der der Anteil an 2,4-Decadienal, bezogen auf das Gesamtgewicht der Aromamischung, 1,0 bis 6 Gew.-% beträgt und/oder in der der Anteil an 2,4-Nonadienal, bezogen auf das Gesamtgewicht der Aromamischung 0,5 bis 2,5 Gew.-% beträgt.

Eine erfindungsgemäße Aromamischung, hergestellt durch ein Verfahren wie vorangehend beschrieben, umfasst das bzw. die Reaktionsprodukt(e) 2,4-Nonadienal und/oder 2,4-Decadienal wie vorangehend definiert. Je nach Wahl der Verfahrensbedingungen werden dabei unterschiedliche Isomerenverhältnisse für 2,4-Nonadienal bzw. 2,4-Decadienal erhalten.

Die erfindungsgemäßen Aromamischungen, hergestellt durch ein Verfahren wie vorangehend beschrieben, sind besonders vorteilhaft, da sie durch die Präsenz beispielsweise weiterer Aldehyde neben dem geruchs- bzw. geschmacksbestimmenden 2,4-Nonadienal und/oder 2,4-Decadienal ein besonders komplexes und authentisches Geruchs- bzw. Geschmacksprofil aufweisen. Sie sind dadurch besonders vorteilhaft in erfindungsgemäßen Zubereitungen und Halbfertigwaren (wie weiter unten beschrieben) einsetzbar. Zudem können die erfindungsgemäßen Aromamischungen im Gegensatz zu vollsynthetisch aus kleineren Bauteilen mittels Kupplungsreaktionen hergestellten ungesättigten Dienalen ohne den Zusatz weiterer Chemikalien (außer Luftsauerstoff) preiswert und umweltfreundlich aus nachwachsenden Rohstoffen gewonnen werden und sind lebensmitteltechnologisch und -rechtlich ohne Bedenken in Nahrungsmitteln einsetzbar.

Gemäß einem weiteren Aspekt betrifft die vorliegende Erfindung eine Zubereitung, vorzugsweise eine der Ernährung oder dem Genuss dienende Zubereitung, oder eine Halbfertigware zur Herstellung besagter Zubereitung, umfassend eine Aromamischung wie vorangehend beschrieben, vorzugsweise in einer Menge, die ausreicht, eine oder mehrere Geruchs- und/oder Geschmacksnoten ausgewählt aus der Gruppe bestehend aus Fleisch, insbesondere Huhn, Lamm oder Rind, fettig, ranzig, nussig, sauer, süß, Gemüse und Obst, bevorzugt Gurke, Melone, Birne, Apfel, Passionsfrucht, Spargel, Tomate, und Dairy, bevorzugt Sourcream, Joghurt, Käse, zu vermitteln, zu modifizieren und/oder zu verstärken.

In einer bevorzugten Ausführungsform der erfindungsgemäßen Zubereitung bzw. Halbfertigware beträgt der Anteil der erfindungsgemäßen Aromamischung, bezogen auf das Gesamtgewicht der Zubereitung bzw. der Halbfertigware, 0,0001 Gew.-% (1 ppm) bis 30 Gew.-% (300000 ppm), bevorzugt 0,0001 Gew.-% (1 ppm) bis 5 Gew.-% (50000 ppm), bevorzugt 0,001 Gew.-% (10 ppm) bis 0,5 Gew.-% (5000 ppm), besonders bevorzugt 0,01 Gew.-% (100 ppm) bis 0,5 Gew.-% (5000 ppm), besonders bevorzugt 0,05 Gew.-% (500 ppm) bis 0,2 Gew.-% (2000 ppm).

Bei den erfindungsgemäßen Zubereitungen handelt es sich bevorzugt um Nahrungsmittel und/oder Nahrungsergänzungsmittel. Bei den erfindungsgemäßen Halbfertigwaren handelt es sich bevorzugt um Halbfertigwaren zur Herstellung von Nahrungsmitteln und/oder Nahrungsergänzungsmitteln.

Gemäß einer bevorzugten Ausführungsform beinhalten die erfindungsgemäßen Aromamischungen oder Zubereitungen oder Halbfertigwaren weitere, vorzugsweise flüchtige, Aromastoffe.

Bevorzugt beinhalten die erfindungsgemäßen Aromamischungen bzw. Zubereitungen bzw. Halbfertigwaren einen oder mehrere Inhaltsstoffe ausgewählt aus der Gruppe bestehend aus flüchtigen organische Säuren, Alkohole, Thiole, Disulfide, heterocyclische Verbindungen (insbesondere Pyridine, Pyrroline, Thiazole und Thiazoline), Aldehyde, Ketone, Ester und Lactone.

Besonders bevorzugte Inhaltsstoffe - wobei beliebige Inhaltsstoffe miteinander auf beliebige Weise kombiniert werden können - sind dabei:
- Organische Säuren:
   Essigsäure, Buttersäure, 2- bzw. 3-Methylbuttersäure, Caprinsäure, Capronsäure, Phenylessigsäure;
- Alkohole:
   Ethanol, Propylenglykol, 1,3-Octenol, cis-3-Hexenol, Linalool, Benzylalkohol, p-Kresol, 2,6-Dimethylthiophenol, Guajacol, Eugenol;
- Disulfide /Thiole:
   Dimethylsulfid, Difurfuryldisulfid, Methylthiopropanal, 2-Methyl-3-methyldithiofuran und Bis(2-methyl-3-furyl)disulfid, Methylfuranthiol, 2-(4-methyl-1,3-thiazol-5-yl)ethanol (Sulfurol), Methyltetrahydrofuranthiol, 3-Methyl-2-buten-1-thiol, 3-Thio-2-methylpentanol, 2-Furfurylthiol, Thiophenol, 2-Methylthiophenol und 2-Mercaptobutanon;
- Pyridine:
   2-Acetylpyridin; Pyrazine, weiter bevorzugt Methylpyrazin, 2,5-Methylethylpyrazin, 2,3,5-Trimethylpyrazin, Acetylpyrazin, 2,3-Diethyl-5- methylpyrazin, 2-Ethyl-3,5-dimethylpyrazin und 2-Isopropyl-3-methoxypyrazin;
- Thiazole/Thiazoline:
   2-Acetylthiazol, 2-Acetyl-2-thiazolin;
- Pyrroline:
   2-Propionyl-1-pyrrolin und 2-Acetyl-1-pyrrolin;
- sonstige heterocyclische Verbindungen:
   Indol, Skatol;
- Aldehyde:
   Acetaldehyd, trans-4,5-Epoxy-(2E)-decenal, cis-4,5-Epoxy-(2E)-decenal, trans-4,5-Epoxy-(2E)-nonenal, cis-4,5-Epoxy-(2E)-nonenal, (E,E)-2,4-Undecadienal, (E,E,Z)-2,4,6-Nonatrienal, (E)-2-Undecenal, (Z)-2-Decenal, (E)-2-Decenal, (E)-2-Nonenal, (Z)-2-Nonenal, (E,Z)-2,6-Nonadienal, 3-Methylthiopropanal (Methional), Vanillin und Phenylacetaldehyd;
- Ketone:
   3,4-Dimethylcyclopentan-1,2-dion, 3-Hydroxy-4,5-dimethylfuran-2(5H)-on (Sotolon), 2-Aminoacetophenon, 3-Hydroxy-4,5-dimethyl-2(5H)-furanon, 2,5-Dimethyl-4-hydroxy-3-[2H]-furanon (Furaneol^{©}), Tetrahydrothiophen-3-on und 3-Thiobutan-2-on;
- Ester und Lactone:
   Methylbutanoat, Ethyl-3-methylbutanoat, Propyl-2-methylbutanoat, (Z)-6-Dodecen-y-lacton, 4-Hydroxy-2-nonensäurelacton, δ-Undecalacton, γ-Nonalacton und γ-Octalacton.

Die erfindungsgemäßen Aromamischungen bzw. Zubereitungen bzw. Halbfertigwaren können gemäß einer bevorzugten Ausführungsform auch noch weitere / andere Aromastoffe umfassen. Der oder die weiteren Aromastoffe können dabei z.B. auch in Form von Reaktionsaromen (Maillard-Produkte), Extrakten bzw. ätherischen Ölen von Pflanzen oder Pflanzenteilen bzw. Fraktionen davon, Raucharomen oder anderen aromagebenden Zubereitungen (z.B. Protein[teil]hydrolysaten), Grillaromen, Pflanzenextrakten, Gewürzen, Gewürzzubereitungen, Gemüsesorten und/oder Gemüsezubereitungen eingesetzt werden.

Insbesondere sind hierfür Aromastoffe oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen.

Der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kakao, Kaffee, Grüntee, Schwarztee, mit (Grün-, Schwarz-) Tee-Extrakten angereicherte (Grün-, Schwarz-)Tee-Getränke, Rooibos-Tee, andere Kräutertees, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Pudding, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, isoliertes oder enzymatisch behandeltes Sojaprotein enthaltende Getränke, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitung zum Süßen oder Weißen von Getränken oder anderen Nahrungsmitteln.

Bei den erfindungsgemäßen Zubereitungen bzw. Halbfertigwaren kann es sich gemäß einer bevorzugten Ausführungsform auch um Tiernahrung bzw. Vorstufen zur Herstellung von Tiernahrung handeln.

Als weitere Bestandteile für erfindungsgemäße Zubereitungen bzw. Halbfertigwaren können übliche Grund-, Hilfs- und Zusatzstoffe für Nahrungs- oder Genussmittel verwendet werden.

Weitere übliche Grund-, Hilfs- und Zusatzstoffe für erfindungsgemäße, vorzugsweise der Ernährung oder dem Genuss dienende, Zubereitungen bzw. Halbfertigwaren können in Mengen von 5 bis 99.999999 Gew.-%, vorzugsweise 10 bis 80 Gew.-%, bezogen auf das Gesamtgewicht der Zubereitung bzw. Halbfertigware, enthalten sein. Ferner können die Zubereitungen bzw. Halbfertigwaren Wasser in einer Menge bis zu 99.999999 Gew.-%, vorzugsweise 5 bis 80 Gew.-%, bezogen auf das Gesamtgewicht der Zubereitung bzw. Halbfertigwaren, aufweisen.

Beispiele üblicher Grund-, Hilfs- und Zusatzstoffe für erfindungsgemäße Zubereitungen bzw. Halbfertigwaren sind Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, Geschmackskorrigenzien für unangenehme Geschmackseindrücke (z. B. Hesperetin, Phloretin oder andere gemäß US 2008/0227867 zu verwendende Hydroxychalkonderivate sowie gegebenenfalls die dort beschriebenen Lactone), Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure, Milchsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam, Stevioside, Rebaudioside, Acesulfam K, Neohesperidindihydrochalkon, Thaumatin, Superaspartam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), ätherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, synthetische, natürliche oder naturidentische Aromastoffe oder Riechstoffe sowie Geruchskorrigenzien.

Ein weiterer Aspekt der vorliegenden Erfindung betrifft die Verwendung einer erfindungsgemäßen Aromamischung wie vorangehend beschrieben zum Vermitteln, Modifizieren und/oder Verstärken einer oder mehrerer Geruchs- und/oder Geschmacksnoten ausgewählt aus der Gruppe bestehend aus Fleisch, insbesondere Huhn, Lamm oder Rind, fettig, ranzig, nussig, sauer, süß, Gemüse und Obst, bevorzugt Gurke, Melone, Birne, Apfel, Passionsfrucht, Spargel, Tomate, und Dairy, bevorzugt Sourcream, Joghurt, Käse, und/oder zur Aromatisierung einer Zubereitung, vorzugsweise einer der Ernährung oder dem Genuss dienenden Zubereitung, oder einer Halbfertigware zur Herstellung besagter Zubereitung, vorzugsweise in einer Menge, die ausreicht, eine oder mehrere Geruchs- und/oder Geschmacksnoten ausgewählt aus der Gruppe bestehend aus Fleisch, insbesondere Huhn, Lamm oder Rind, fettig, ranzig, nussig, sauer, süß, Gemüse und Obst, bevorzugt Gurke, Melone, Birne, Apfel, Passionsfrucht, Spargel, Tomate, und Dairy, bevorzugt Sourcream, Joghurt, Käse, zu vermitteln, zu modifizieren und/oder zu verstärken.

Die hierin beschriebenen Ausführungsformen des erfindungsgemäßen Verfahrens entsprechen oder können abgeleitet werden von vorangehend beschriebenen Ausführungsformen der erfindungsgemäßen Aromamischungen, Zubereitungen und Verwendungen und umgekehrt. Die hierin beschriebenen Ausführungsformen können des Weiteren, soweit für den Fachmann technisch sinnvoll, beliebig miteinander kombiniert werden.

Die vorliegende Erfindung wird im Folgenden anhand von Beispielen näher beschrieben. Die in den Beispielen angegebenen Prozentangaben sind relative Prozente, die per Gaschromatographie bestimmt wurden. Ein Korrekturfaktor für Triacetin wurde nicht verwendet.

### Herstellung der Aromamischungen

### Beispiel 1: Umsetzung von Granatapfelkernöl

10 g Granatapfelkernöl werden bei 120 °C mit Luft (20 L/h) 6 h lang begast. Anschließend wird das Reaktionsgemisch 1 h auf 170 °C erhitzt. Anschließend werden 8,5 g dieser Rohware mit 0,8 g Triacetin versetzt und das gewünschte Produkt anschließend zusammen mit dem Triacetin abdestilliert (Bedingungen: 0,5 mbar, 180 °C, 1 h).

Nach destillativer Aufreinigung enthält die erhaltene Lösung (0,83 g) 96,04% Triacetin, 0,22% 2E,4Z-Nonadienal sowie 0,25% 2E,4E-Nonadienal (nicht erfindungsgemäße Aromamischung).

Evaluierung (Geruch): Lammfleisch, fett, ranzig.

### Vergleichsbeispiel 2: Umsetzung von Granatapfelkernöl

11 g Granatapfelkernöl werden bei 50 °C mit Luft (20 L/h) 6 h lang begast. Anschließend wird das Reaktionsgemisch 1 h auf 170 °C erhitzt. Anschließend werden 10,0 g dieser Rohware mit 1,0 g Triacetin versetzt und das gewünschte Produkt anschließend zusammen mit dem Triacetin abdestilliert (Bedingungen: 0,5 mbar, 180 °C, 1 h).

Nach destillativer Aufreinigung enthält die erhaltene Lösung (1,01 g) 96,50% Triacetin, 0.22% 2E,4Z-Nonadienal sowie 0,07% 2E,4E-Nonadienal (nicht erfindungsgemäße Aromamischung).

Evaluierung (Geruch): fettig, sauer, süß, Lammfleisch.

### Beispiel 3: Umsetzung von Granatapfelkernöl

11 g Granatapfelkernöl werden bei 170 °C mit Luft (20 L/h) 6 h lang begast. Anschließend werden 10,0 g dieser Rohware mit 1,0 g Triacetin versetzt und das gewünschte Produkt anschließend zusammen mit dem Triacetin abdestilliert (Bedingungen: 0,5 mbar, 180 °C, 1 h).

Nach destillativer Aufreinigung enthält die erhaltene Lösung (1,03 g) 96,50% Triacetin, 0,12% 2E,4Z-Nonadienal sowie 0,27% 2E,4E-Nonadienal (nicht erfindungsgemäße Aromamischung).

Evaluierung (Geruch): Lammfleisch, fettig, ranzig.

### Beispiel 4: Umsetzung von Granatapfelkernöl

10 g Granatapfelkernöl werden bei 170 °C mit Luft (20 L/h) 6 h lang begast. Anschließend wird das Reaktionsgemisch 1 h auf 170 °C erhitzt. Anschließend werden 9,5 g dieser Rohware mit 0,9 g Triacetin versetzt und das gewünschte Produkt anschließend zusammen mit dem Triacetin an der Kugelrohrdestille fraktioniert destilliert.
Fraktion 1 (0,5 mbar, 90°C, 0,5 h) enthält 98,22% Triacetin, 0,04% 2E,4Z-Nonadienal sowie 0,03% 2E,4E-Nonadienal (nicht erfindungsgemäße Aromamischung).
   Evaluierung Geruch (Fraktion 1): sauer, Olivennote, fruchtig, leicht grün
Fraktion 2 (0,5 mbar, 130°C, 0,5 h) enthält 96,55% Triacetin, 0,42% 2E,4Z-Nonadienal sowie 0,50% 2E,4E-Nonadienal (erfindungsgemäße Aromamischung).
   Evaluierung Geruch (Fraktion 2): fettig, Lamm, leicht ranzig
Fraktion 3 (0,5 mbar, 160°C, 0,5 h) enthält 92,93% Triacetin, 0,30% 2E,4Z-Nonadienal sowie 0,51% 2E,4E-Nonadienal (erfindungsgemäße Aromamischung).
   Evaluierung Geruch (Fraktion 3): Lammfleisch, fettig, leicht nussig.

Dieses Beispiel zeigt, dass durch Abtrennen der leichter flüchtigen Komponenten über eine fraktionierte Destillation der sensorische Wert dieser Verbindungen deutlich gesteigert wird.

### Beispiel 5: Umsetzung von Sonnenblumenöl

1,0 kg Sonnenblumenöl wird bei 120 °C mit Luft (32 L/h) 6 h lang begast. Anschließend wird das Reaktionsgemisch für 2h auf 150 °C sowie anschließend für 0,5 h auf 170 °C erhitzt. Anschließend wird dieser Ansatz mit 56,0 g Triacetin versetzt und das gewünschte Produkt anschließend zusammen mit dem Triacetin abdestilliert (Bedingungen: 0,2 mbar, Innentemperatur: 175 °C).

Nach destillativer Aufreinigung enthält die erhaltene Lösung (34,8 g) 87,7 % Triacetin, 4,26% 2E,4E-Decadienal sowie 0,88% 2E,4Z-Decadienal (erfindungsgemäße Aromamischung).

### Einsatz der vorangehend hergestellten Aromamischungen in Halbfertigwaren bzw. Zubereitungen

### Anwendungsbeispiel 1: Huhnaroma

| **Bestandteil** | **(g)** |
|---|---|
| Acetylmethylcarbinol | 0,20 |
| Buttersäure | 2,00 |
| Capronsäure | 1,00 |
| Caprylsäure | 1,00 |
| 2,4-Decadienal (Beispiel 5) | 16,00 |
| Decenal-tr.2 | 0,20 |
| Dimethyloxyfuron/Sotolon (1%ige Lsg.) | 0,40 |
| Furaneol (10%ige Lsg.) | 20,00 |
| Indol (1%ige Lsg.) | 0,40 |
| 2,3-Methylfuranthiol | 0,80 |
| Methyltetrahydrofuranthiol | 0,30 |
| Methylthiopropanal-3 | 0,15 |
| 2,4-Nonadienal (analog Beispiel 4, Fraktion 2) | 30,00 |
| Thiobutanon-3,2 | 1,00 |
| Pflanzenöl-Triglyzeride | 926,55 |
| Summe | 1000,00 |

Diese Aromakomposition wird in nachfolgend beschriebenen Anwendungsbeispielen eingesetzt.

### Anwendungsbeispiel 2: Sprühgetrocknete Aromakomposition

Es wird - ausgehend von dem zuvor beschriebenen Anwendungsbeispiel 1 - eine sprühgetrocknete Aromakomposition hergestellt, die nachfolgend weiter eingesetzt wird:

| **Inhaltsstoff** | **A** |
|---|---|
| Capsul | 200 g |
| Maltodextrin | 600 g |
| Aromakomposition Zusammensetzung Anwendungsbeispiel 1 | 200 g |
| Wasser | 1000 g |

Die Bestandteile werden in demineralisiertem Wasser gelöst und anschließend sprühgetrocknet. Diese werden in den nachfolgenden Anwendungsbeispielen verwendet.

### Anwendungsbeispiel 3: Instantsuppe, Typ Hühnersuppe mit Nudeln

A = Vergleichszubereitung
B = erfindungsgemäße Zubereitung

| **Inhaltsstoff** | **A** | **B** |
|---|---|---|
| Stärke | 16 g | 16 g |
| Kochsalz | 7 g | 7 g |
| Saccharose, raffiniert | 3,2 g | 3,2 g |
| Natriumglutamat | 3,2 g | 3,2 g |
| Natriuminosinat / Natriumguanylat im Verhältnis 1:1 | 0,8 g | 0,8 g |
| Säurehydrolysiertes Pflanzenprotein | 8,0 g | 8,0 g |
| Fettpulver | 2,0 g | 2,0 g |
| Gemüsefett, sprühgetrocknet | 1,0 g | 1,0 g |
| Gefriergetrocknetes Hühnerfleisch, in Stückchen | 2,15 g | 2,15 g |
| Suppen-Nudeln | 32,0 g | 32,0 g |
| Maltodextrin | 12,0 g | 7,6 g |
| Chinesisches Gemüse, gefriergetrocknet | 4,6 g | 4,6 g |
| Huhnaroma | 8,0 g | 6,9 g |
| Lebensmittelfarbstoff Riboflavin | 0,05 g | 0,05 g |
| Sprühgetrocknete Aromakomposition aus Anwendungsbeispiel 2 | -/- | 5,5 g |

4,6 g der Pulvermischung werden 10 Minuten in 100 ml Wasser gekocht, um eine verzehrfertige Suppe zu erhalten. Die erfindungsgemäße Zubereitung B wird in Bezug auf erwünschte fettige Aromen erinnernd an Hähnchenhaut als stärker und lang anhaltender bewertet als Zubereitung A, was ein deutlich authentischeres Profil ergibt.

### Anwendungsbeispiel 4: Bouillon

A = Vergleichszubereitung
B = erfindungsgemäße Zubereitung

| **Inhaltsstoff** | **A** | **B** |
|---|---|---|
| Fettpulver | 8,77 g | 8,77 g |
| Natriumglutamat | 8,77 g | 8,77 g |
| Hefeextrakt Pulver | 12,28 g | 12,28 g |
| Kochsalz | 29,83 g | 29,83 g |
| Maltodextrin | 37,28 g | 20,78 g |
| Natürlicher Gemüseextrakt | 3,07 g | 3,07 g |
| Sprühgetrocknete Aromakomposition aus Anwendungsbeispiel 2 | -/- | 16,5 g |

15 g der Pulvermischung werden mit 1000 ml heißem Wasser aufgegossen. Bei der Verkostung durch ein Panel ausgebildeter Testpersonen wird die erfindungsgemäße Zubereitung B hinsichtlich ihres Aroma und Geschmack als deutlich reicher, ausgewogener, kräftiger und langanhaltender als die Vergleichszubereitung A bewertet.

### Anwendungsbeispiel 5: Weiße Soße

A = Vergleichszubereitung
B = erfindungsgemäße Zubereitung

| **Bestandteil** | **A** | **B** |
|---|---|---|
| Maltodextrin | 26,00 g | 23,25 g |
| Kochsalz | 7,50 g | 7,50 g |
| Natriumglutamat | 2,00 g | 2,00 g |
| Pflanzenfett | 5,00 g | 5,00 g |
| Pfeffer, weiß | 0,02 g | 0,02 g |
| Zwiebelpulver | 1,48 g | 1,48 g |
| vorverkleisterte Maisstärke | 30,00 g | 30,00 g |
| Fettpulver | 28,00 g | 28,00 g |
| Sprühgetrocknete Zusammensetzung Anwendungsbeispiel 2 | -/- | 2,75 g |

90 g der Soßenmischung werden mit 1000 ml heißem Wasser aufgegossen und mit dem Schneebesen kräftig verrührt. Bei der Verkostung durch ein Panel ausgebildeter Testpersonen wird die erfindungsgemäße Zubereitung B hinsichtlich ihres Aromas und Geschmacks als deutlich reicher, ausgewogener, kräftiger und langanhaltender als die Vergleichszubereitung A bewertet.

### Anwendungsbeispiel 6: Braune Soße

A = Vergleichszubereitung
B = erfindungsgemäße Zubereitung

| **Bestandteil** | **A** | **B** |
|---|---|---|
| Stärke | 40,00 g | 40,00 g |
| Maltodextrin | 33,10 g | 30,35 g |
| Kochsalz | 6,00 g | 6,00 g |
| Zuckerkulör, sprühgetrocknet | 5,00 g | 5,00 g |
| Hefeextraktpulver | 3,00 g | 3,00 g |
| Natriumglutamat | 2,00 g | 2,00 g |
| Zucker | 0,50 g | 0,50 g |
| Fettpulver | 5,00 g | 5,00 g |
| Tomatenpulver | 3,00 g | 3,00 g |
| Natürlicher Gemüseextrakt | 1,00 g | 1,00 g |
| Zwiebelextrakt | 0,30 g | 0,30 g |
| Pfefferextrakt | 0,10 g | 0,10 g |
| Trockenaroma | 1,00 g | 1,00 g |
| Sprühgetrocknete Zusammensetzung Anwendungsbeispiel 2 | -/- | 2,75 g |

90 g der Soßenmischung werden mit 1000 ml heißem Wasser aufgegossen und mit dem Schneebesen kräftig verrührt. Bei der Verkostung durch ein Panel ausgebildeter Testpersonen wird die erfindungsgemäße Zubereitung B hinsichtlich ihres Aromas und Geschmacks als deutlich reicher, ausgewogener, kräftiger und langanhaltender als die Vergleichszubereitung A bewertet.

### Anwendungsbeispiel 7: Würzmischung für Kartoffelchips

A = Vergleichszubereitung
B = erfindungsgemäße Zubereitung

| **Bestandteil** | **A** | **B** |
|---|---|---|
| Natriumglutamat | 3,50 g | 3,50 g |
| Käsepulver | 10,00 g | 10,00 g |
| Knoblauchpulver | 2,00 g | 2,00 g |
| Molkenpulver | 38,86 g | 36,86 g |
| Würzextraktöl | 0,20 g | 0,20 g |
| Paprikapulver | 9,80 g | 9,80 g |
| Kochsalz | 21,00 g | 19,00 g |
| Tomatenpulver | 9,00 g | 9,00 g |
| Trockenaroma | 2,50 g | 2,50 g |
| Siliciumdioxid | 0,02 g | 0,02 g |
| Pflanzenöl | 0,02 g | 0,02 g |
| Zwiebelpulver | 3,00 g | 3,00 g |
| Sahne Aromakonzentrat | 0,03 g | 0,03 g |
| Käse Aroma | 0,03 g | 0,03 g |
| Tomaten Aromakonzentrat | 0,04 g | 0,04 g |
| Sprühgetrocknete Zusammensetzung Anwendungsbeispiel 2 | -/- | 4,00 g |

6 g der Würzmischung werden auf 94 g Kartoffelchips aufgezogen.

### Anwendungsbeispiel 8: Hähnchenfleischwürzmischung für (Fertig)-Nudeln

| **Inhaltsstoff** | **Gew.-%** |
|---|---|
| Hähnchenaroma | 5,00 |
| Zuckercouleur | 3,00 |
| Zitronensäure (wasserfrei) | 0,40 |
| Schnittlauch (entwässert) | 2,00 |
| Maltodextrin (ex Tapoica) | 5,30 |
| Mononatriumglutamat | 15,00 |
| Zwiebelpulver | 5,00 |
| Ribotide | 0,80 |
| Natriumchlorid | 39,20 |
| Zucker | 2,80 |
| Süßmolkepulver | 6,50 |
| Sprühgetrocknete Zusammensetzung Anwendungsbeispiel 2 | 15,00 |

Alle Inhaltsstoffe werden vermischt, bis sich eine homogene Mischung ergibt.

### Anwendungsbeispiel 9: Birnenaroma

| **Bestandteil** | **(g)** |
|---|---|
| Aldehyd C6 (Hexanal) | 0,20 |
| Alkohol C6 (Hexanol) | 1,00 |
| 2,4-Nonadienal (Vergleichsbeispiel 2) | 0,30 |
| 2,4-Decadienal (Beispiel 5) | 0,02 |
| Butylacetat | 30,00 |
| Ethylbutyrat | 2,00 |
| Ethyldecadienoat tr., cis 2,4 | 4,00 |
| Hexenylacetat, cis 3 | 1,00 |
| Hexenylacetat, trans 2 | 2,00 |
| Hexylacetat | 35,00 |
| Propylenglycol | 924,48 |
| Summe | 1000,00 |

Es werden typischerweise 10 g des hier beschriebenen Aromas pro 100 L Fertiggetränk eingesetzt.

### Anwendungsbeispiel 10: Passionsfruchtaroma (nicht erfindungsgemäß)

| **Bestandteil** | **(g)** |
|---|---|
| Nonalacton, gamma | 3,00 |
| Decalacton, gamma | 13,00 |
| Decadienal-2,4 tr.tr. (0,1 %ige Lsg.) | 0,08 |
| Ethylbutyrat | 13,00 |
| Ethylcapronat | 13,00 |
| Furfural | 12,00 |
| Geraniol | 0,30 |
| Ionon, beta | 3,00 |
| Isovaleraldehyd | 0,20 |
| 2,4-Nonadienal (Beispiel 1) | 1,20 |
| Thiomentanon-8,3 | 0,13 |
| Phenylethylalkohol | 6,50 |
| Terpineol alpha | 10,00 |
| Propylenglykol | 924,59 |
| Summe | 1000,00 |

Es werden typischerweise 10 g des hier beschriebenen Aromas pro 100 L Fertiggetränk eingesetzt.

### Anwendungsbeispiel 11: Pilzaroma

| **Bestandteil** | **(g)** |
|---|---|
| Octanol-1 | 4,00 |
| Caprylsäure | 15,00 |
| 2,4-Decadienal (Beispiel 5) | 60,00 |
| Decalacton, delta | 7,00 |
| 2,4-Nonadienal (Beispiel 4, Fraktion 2) | 200,00 |
| Octenol-1,3 | 50,00 |
| Octenon-1,3 | 3,00 |
| Triacetin | 661,00 |
| Summe | 1000,00 |

Es werden typischerweise 10 g des hier beschriebenen Aromas für 100 kg Suppe benötigt.

## Patentansprüche

1. Verfahren zur Herstellung einer Aromamischung enthaltend ein oder mehrere ungesättigte(s) Dienal(e), bestehend aus oder umfassend die Schritte
a) Luftoxidation eines oder mehrerer Edukte(s) umfassend oder bestehend aus einem oder mehreren Stoff(en) ausgewählt aus der Gruppe bestehend aus Punicinsäure, Punicinsäureester, Elaeostearinsäure, Elaeostearinsäureester, Linolsäure und Linolsäureester,
b) optional Zersetzung eines Teils oder aller oder im Wesentlichen aller Peroxide in der in Schritt a) erhaltenen Mischung,
c) Aufreinigung bzw. Aufkonzentrierung des/der in Schritt a) entstandenen ungesättigten Dienals/Dienale aus bzw. in der in Schritt a) und/oder b), falls vorhanden, erhaltenen Mischung,
wobei die Luftoxidation in Schritt a) bei einer Temperatur von 70 bis 200°C, vorzugsweise von 80 bis 200°C, bevorzugt von 90 bis 170°C, weiter bevorzugt von 100 bis 130°C, durchgeführt wird.

2. Verfahren nach Anspruch 1, wobei nach Aufreinigung bzw. Aufkonzentrierung in Schritt c) ein Rückstand erhalten wird, der eine Restmenge an Edukt(en) enthält und wobei das Verfahren zusätzlich folgenden Schritt umfasst:
d) Wiederholen, bevorzugt mehrmaliges Wiederholen, der in Anspruch 1 definierten Schritte, wobei der bei der Aufreinigung bzw. Aufkonzentrierung in Schritt c) erhaltene Rückstand in Schritt a) eingesetzt wird.

3. Verfahren nach Anspruch 1 oder 2, wobei Schritt a) in Gegenwart eines oder mehrerer Lösungsmittel(s) durchgeführt wird, vorzugsweise wobei das bzw. ein oder zwei bzw. die Lösungsmittel Triacetin und/oder Triethylcitrat ist bzw. sind, besonders bevorzugt wobei insgesamt 2 bis 20 Gew.-%, weiter bevorzugt 5 bis 15 Gew.-%, jeweils bezogen auf das Gesamtgewicht des/der Edukte(s), an Lösungsmittel(n), vorzugsweise an Triacetin und/oder Triethylcitrat, eingesetzt werden.

4. Verfahren nach einem der Ansprüche 1 bis 3, wobei für die Luftoxidation in Schritt a) eine Luftmenge von 10 bis 1000 l/h, vorzugsweise 30 bis 500 l/h, besonders bevorzugt 40 bis 100 l/h, pro kg Edukt(e) eingesetzt wird.

5. Verfahren nach einem der Ansprüche 1 bis 4, wobei die Luftoxidation in Schritt a) über einen Zeitraum von 1 bis 30 h, bevorzugt 2 bis 12 h, besonders bevorzugt von 4 bis 12 h, durchgeführt wird.

6. Verfahren nach einem der Ansprüche 1 bis 5, wobei in Schritt b), falls vorhanden, die Zersetzung eines Teils oder aller oder im Wesentlichen aller Peroxide in der in Schritt a) erhaltenen Mischung, vorzugsweise durch Erhitzen, bevorzugt auf Temperaturen von 100 bis 200°C, stattfindet, vorzugsweise über einen Zeitraum von 0,5 bis 5 h.

7. Verfahren nach einem der Ansprüche 1 bis 6, wobei die Aufreinigung bzw. Aufkonzentrierung in Schritt c) mittels Destillation, vorzugsweise mittels fraktionierter Destillation, stattfindet.

8. Verfahren nach einem der Ansprüche 1 bis 7, wobei vor Beginn der Aufreinigung bzw. Aufkonzentrierung in Schritt c) ein oder mehrere Lösungsmittel, bevorzugt Triacetin, zugegeben wird bzw. werden, vorzugsweise in einer Gesamtmenge von 1 bis 60 Gew.-%, bevorzugt von 1 bis 40 Gew.-%, besonders bevorzugt von 2 bis 20 Gew.-%, jeweils bezogen auf das Gesamtgewicht des/der Edukte(s).

9. Verfahren nach einem der Ansprüche 1 bis 8, wobei vor Beginn der Aufreinigung bzw. Aufkonzentrierung in Schritt c), vorzugsweise vor Beginn der Destillation, ein Waschschritt mit basischer, wässriger Lösung erfolgt.

10. Aromamischung, hergestellt durch ein Verfahren nach einem der Ansprüche 1 bis 9,
in der das Gewichtsverhältnis von 2E/4E-Decadienal zu 2E/4Z-Decadienal 4:1 bis 5:1 beträgt und/oder in der der Anteil an 2,4-Decadienal, bezogen auf das Gesamtgewicht der Aromamischung, 1,0 bis 6 Gew.-% beträgt und/oder in der der Anteil an 2,4-Nonadienal, bezogen auf das Gesamtgewicht der Aromamischung 0,5 bis 2,5 Gew.-% beträgt.

11. Zubereitung, vorzugsweise der Ernährung oder dem Genuss dienende Zubereitung, oder Halbfertigware zur Herstellung besagter Zubereitung, umfassend eine Aromamischung nach Anspruch 10, vorzugsweise in einer Menge, die ausreicht, eine oder mehrere Geruchs- und/oder Geschmacksnoten ausgewählt aus der Gruppe bestehend aus Fleisch, insbesondere Huhn, Lamm oder Rind, fettig, ranzig, nussig, sauer, süß, Gemüse und Obst, bevorzugt Gurke, Melone, Birne, Apfel, Passionsfrucht, Spargel, Tomate, und Dairy, bevorzugt Sourcream, Joghurt, Käse, zu vermitteln, zu modifizieren und/oder zu verstärken.

12. Zubereitung bzw. Halbfertigware nach Anspruch 11, wobei der Anteil der Aromamischung, bezogen auf das Gesamtgewicht der Zubereitung bzw. der Halbfertigware, 0,0001 bis 30 Gew.-%, bevorzugt 0,0001 bis 5 Gew.-%, bevorzugt 0,001 bis 0,5 Gew.-%, besonders bevorzugt 0,01 bis 0,5 Gew.-%, besonders bevorzugt 0,05 bis 0,2 Gew.-%, beträgt.

13. Verwendung einer Aromamischung nach Anspruch 10 zum Vermitteln, Modifizieren und/oder Verstärken einer oder mehrerer Geruchs- und/oder Geschmacksnoten ausgewählt aus der Gruppe bestehend aus Fleisch, insbesondere Huhn, Lamm oder Rind, fettig, ranzig, nussig, sauer, süß, Gemüse und Obst, bevorzugt Gurke, Melone, Birne, Apfel, Passionsfrucht, Spargel, Tomate, und Dairy, bevorzugt Sourcream, Joghurt, Käse, und/oder zur Aromatisierung einer Zubereitung, vorzugsweise einer der Ernährung oder dem Genuss dienenden Zubereitung, oder einer Halbfertigware zur Herstellung besagter Zubereitung, vorzugsweise in einer Menge, die ausreicht, eine oder mehrere Geruchs- und/oder Geschmacksnoten ausgewählt aus der Gruppe bestehend aus Fleisch, insbesondere Huhn, Lamm oder Rind, fettig, ranzig, nussig, sauer, süß, Gemüse und Obst, bevorzugt Gurke, Melone, Birne, Apfel, Passionsfrucht, Spargel, Tomate, und Dairy, bevorzugt Sourcream, Joghurt, Käse, zu vermitteln, zu modifizieren und/oder zu verstärken.

## Claims

1. Process for the preparation of an aroma mixture containing one or more unsaturated dienal(s), consisting of or comprising the steps of
a) air oxidation of one or more educt(s) comprising or consisting of one or more substance(s) selected from the group consisting of punicic acid, punicic acid ester, elaeostearic acid, elaeostearic acid ester, linoleic acid, and linoleic acid ester,
b) optionally decomposing a part or all or substantially all of the peroxides in the mixture obtained in step a),
c) purification or concentration of the unsaturated dienal(s) formed in step a) from or in the mixture obtained in step a) and/or b), if present,
wherein the air oxidation in step a) is carried out at a temperature of from 70 to 200°C, preferably from 80 to 200°C, preferably from 90 to 170°C, more preferably from 100 to 130°C.

2. Process according to claim 1, wherein after purification or concentration in step c) a residue is obtained which contains a residual amount of educt(s) and wherein the process additionally comprises the following step:
(d) Repeating, preferably several times, the steps defined in claim 1, wherein the residue obtained in the purification or concentration in step c) is used in step a).

3. Process according to claim 1 or 2, wherein step a) is carried out in the presence of one or more solvent(s), preferably wherein the or one solvent or two or the solvents is/are triacetin and/or triethyl citrate, particularly preferably wherein a total of 2 to 20% by weight, more preferably 5 to 15% by weight, in each case based on the total weight of the educt(s), of solvent(s), preferably of triacetin and/or triethyl citrate, is/are used.

4. Process according to any one of the claims 1 to 3, wherein for the air oxidation in step a) an air quantity of 10 to 1000 l/h, preferably 30 to 500 l/h, particularly preferably 40 to 100 l/h, per kg of educt(s) is used.

5. Process according to any one of the claims 1 to 4, wherein the air oxidation in step a) is carried out over a period of 1 to 30 h, preferably 2 to 12 h, particularly preferably 4 to 12 h.

6. Process according to any one of the claims 1 to 5, wherein in step b), if present, the decomposition of a part or all or substantially all of the peroxides in the mixture obtained in step a) takes place, preferably by heating, preferably to temperatures of 100 to 200°C, preferably over a period of 0.5 to 5 h.

7. Process according to any one of the claims 1 to 6, wherein the purification or concentration in step c) takes place by distillation, preferably by fractional distillation.

8. Process according to any one of the claims 1 to 7, wherein one or more solvents, preferably triacetin, are added before the start of the purification or concentration in step c), preferably in a total amount of from 1 to 60% by weight, preferably from 1 to 40% by weight, particularly preferably from 2 to 20% by weight, in each case based on the total weight of the educt(s).

9. Process according to any one of the claims 1 to 8, wherein a washing step with basic, aqueous solution is carried out before the start of the purification or concentration in step c), preferably before the start of the distillation.

10. Aroma mixture, prepared by a process according to any one of the claims 1 to 9,
in which the weight ratio of 2E/4E-decadienal to 2E/4Z-decadienal is from 4:1 to 5:1 and/or in which the proportion of 2,4-decadienal, based on the total weight of the aroma mixture, is from 1.0 to 6% by weight and/or in which the proportion of 2,4-nonadienal, based on the total weight of the aroma mixture, is from 0.5 to 2.5% by weight.

11. A preparation, preferably a preparation for nutrition or consumption, or a semi-finished product for the production of said preparation, comprising an aroma mixture according to claim 10, preferably in an amount sufficient to impart, modify and/or enhance one or more odour and/or taste notes selected from the group consisting of meat, in particular chicken, lamb or beef, fatty, rancid, nutty, sour, sweet, vegetables and fruit, preferably cucumber, melon, pear, apple, passion fruit, asparagus, tomato, and dairy, preferably sour cream, yoghurt, cheese.

12. Preparation or semi-finished product according to claim 11, wherein the proportion of the aroma mixture, based on the total weight of the preparation or semi-finished product, is 0.0001 to 30% by weight, preferably 0.0001 to 5% by weight, preferably 0.001 to 0.5% by weight, particularly preferably 0.01 to 0.5% by weight, particularly preferably 0.05 to 0.2% by weight.

13. Use of an aroma mixture according to claim 10 for imparting, modifying and/or enhancing one or more odour and/or taste notes selected from the group consisting of meat, in particular chicken, lamb or beef, fatty, rancid, nutty, sour, sweet, vegetables and fruit, preferably cucumber, melon, pear, apple, passion fruit, asparagus, tomato, and dairy, preferably sour cream, yoghurt, cheese, and/or for flavouring a preparation, preferably a preparation for nutrition or consumption, or a semi-finished product for the production of said preparation, preferably in an amount sufficient to impart, modify and/or enhance one or more odour and/or taste notes selected from the group consisting of meat, in particular chicken, lamb or beef, fatty, rancid, nutty, sour, sweet, vegetables and fruit, preferably cucumber, melon, pear, apple, passion fruit, asparagus, tomato, and dairy, preferably sour cream, yoghurt, cheese.

## Revendications

1. Procédé de production d'un mélange d'arômes contenant un ou plusieurs diénal(s) insaturé(s), constitué des ou comprenant les étapes consistant à
a) oxyder à l'air un ou plusieurs éduit(s) comprenant ou constitués d'une ou de plusieurs substance(s) choisie(s) dans le groupe constitué de l'acide punicique, de l'ester d'acide punicique, de l'acide élaéostéarique, de l'ester de l'acide éléostéarique, de l'acide linoléique et de l'ester de l'acide linoléique,
b) en option décomposer une partie ou la totalité ou pour l'essentiel la totalité des peroxydes dans le mélange obtenu à l'étape a),
c) purifier ou bien concentrer le(s) diénal(s) insaturé(s) obtenu(s) à l'étape a), à partir du ou bien dans le mélange obtenu à l'étape a) et/ou b), si elle est présente,
dans lequel l'oxydation à l'air à l'étape a) est réalisée à une température comprise entre 70 et 200 °C, de préférence entre 80 et 200 °C, de préférence entre 90 et 170 °C, plus préférablement entre 100 et 130 °C.

2. Procédé selon la revendication 1, dans lequel, après la purification ou bien la concentration à l'étape c), on obtient un résidu qui contient une quantité résiduelle d'éduit(s) et dans lequel le procédé comprend en outre l'étape suivante consistant à:
d) répéter, de préférence répéter plusieurs fois, les étapes définies à la revendication 1, dans lequel le résidu obtenu lors de la purification ou bien de la concentration à l'étape c) est utilisé à l'étape a).

3. Procédé selon la revendication 1 ou 2, dans lequel l'étape a) est mise en oeuvre en présence d'un ou de plusieurs solvant(s), de préférence dans lequel le ou bien un ou deux ou bien les solvant(s) est ou bien sont la triacétine et/ou le citrate de triéthyle, de manière particulièrement préférée dans lequel dans l'ensemble 2 à 20 % en poids, plus préférablement 5 à 15 % en poids, respectivement par rapport au poids total du ou des éduit(s), de solvant(s), de préférence de triacétine et/ou de citrate de triéthyle, sont utilisés.

4. Procédé selon l'une quelconque des revendications 1 à 3, dans lequel, pour l'oxydation à l'air à l'étape a), on utilise une quantité d'air allant de 10 à 1000 l/h, de préférence de 30 à 500 l/h, de manière particulièrement préférée de 40 à 100 l/h, par kg d'éduit(s).

5. Procédé selon l'une quelconque des revendications 1 à 4, dans lequel l'oxydation à l'air à l'étape a) est mise en oeuvre pour une durée de 1 à 30 heures, de préférence de 2 à 12 heures, de manière particulièrement préférée de 4 à 12 heures.

6. Procédé selon l'une quelconque des revendications 1 à 5, dans lequel à l'étape b), si elle est présente, la décomposition d'une partie ou de l'ensemble ou pour l'essentiel de l'ensemble des peroxydes dans le mélange obtenu à l'étape a), se fait de préférence par chauffage, de préférence à des températures comprises entre 100 et 200 °C, de préférence pour une durée de 0,5 à 5 heure(s).

7. Procédé selon l'une quelconque des revendications 1 à 6, dans lequel la purification ou bien la concentration à l'étape c) a lieu au moyen de distillation, de préférence au moyen de distillation fractionnée.

8. Procédé selon l'une quelconque des revendications 1 à 7, dans lequel, avant le début de la purification ou bien de la concentration à l'étape c), un ou plusieurs solvant(s), de préférence de la triacétine, est ou bien sont ajouté(s), de préférence en une quantité totale de 1 à 60 % en poids, de préférence de 1 à 40 % en poids, de manière particulièrement préférée de 2 à 20 % en poids, respectivement par rapport au poids total de l'éduit/des éduits.

9. Procédé selon l'une quelconque des revendications 1 à 8, dans lequel, avant le début de la purification ou bien de la concentration à l'étape c), de préférence avant le début de la distillation, une étape de lavage avec une solution aqueuse basique a lieu.

10. Mélange d'arômes produit par un procédé selon l'une quelconque des revendications 1 à 9,
dans lequel le rapport pondéral du 2E/4E-décadiénal au 2E/4Z-décadiénal est de 4:1 à 5:1 et/ou dans lequel la proportion de 2,4-décadiénal, par rapport au poids total du mélange d'arômes, est 1,0 à 6 % en poids et/ou dans lequel la proportion de 2,4-nonadiénal, par rapport au poids total du mélange d'arômes, est de 0,5 à 2,5 % en poids.

11. Préparation, de préférence préparation destinée à l'alimentation ou au plaisir, ou produit semi-fini pour la réalisation de ladite préparation, comprenant un mélange d'arômes selon la revendication 10, de préférence en quantité qui est suffisante pour conférer, modifier et/ou intensifier une ou plusieurs note(s) olfactive(s) et/ou note(s) gustative(s) choisie(s) dans le groupe constitué de viande, en particulier de poulet, d'agneau ou de boeuf, grasse, rance, aux noisettes, aigre, sucrée, de légumes et de fruits, de préférence de concombre, de melon, de poire, de pomme, de fruit de la passion, d'asperges, de tomates et de produits laitiers, de préférence de crème sure, de yaourt, de fromage.

12. Préparation ou bien produit semi-fini selon la revendication 11, dans lequel la proportion du mélange d'arômes, par rapport au poids total de la préparation ou bien du produit semi-fini, est de 0,0001 à 30 % en poids, de préférence de 0,0001 à 5 % en poids, de préférence de 0,001 à 0,5 % en poids, de manière particulièrement préférée de 0,01 à 0,5 % en poids, de manière particulièrement préférée de 0,05 à 0,2 % en poids.

13. Utilisation d'un mélange d'arômes selon la revendication 10 pour conférer, modifier et/ou intensifier une ou plusieurs note(s) olfactive(s) et/ou note(s) gustative(s) choisie(s) dans le groupe constitué de viande, en particulier de poulet, d'agneau ou de boeuf, grasse, rance, aux noisettes, aigre, sucré, de légumes et de fruits, de préférence de concombre, de melon, de poire, de pomme, de fruit de la passion, d'asperges, de tomates, et produits laitiers, de préférence de crème sure, de yaourt, de fromage, et/ou pour aromatiser une préparation, de préférence une préparation destinée à l'alimentation ou au plaisir, ou un produit semi-fini pour la réalisation de ladite préparation, de préférence en une quantité qui est suffisante pour conférer, modifier et/ou intensifier une ou plusieurs note(s) olfactive(s) et/ou note(s) gustative(s) choisie(s) dans le groupe constitué de viande, en particulier de poulet, d'agneau ou de boeuf, grasse, rance, aux noisettes, aigre, sucré, de légumes et de fruits, de préférence de concombre, de melon, de poire, de pomme, de fruit de la passion, d'asperges, de tomates et de produits laitiers, de préférence de crème sure, de yaourt, de fromage.
